# EUROPEAN PATENT SPECIFICATION

(11) **EP 1 812 470 B1**
(45) Date of publication and mention of the grant of the patent: **13.05.2009**
(21) Application number: 05799600.1
(22) Date of filing: 24.10.2005
(51) Int. Cl.: C07K 14/655

(54) **CYCLIZATION OF A PEPTIDE**
CYCLISIERUNG EINES PEPTIDS
CYCLISATION D'UN PEPTIDE

(30) Priority: 01.11.2004 EP 04025866; 18.04.2005 EP 05008380
(43) Date of publication of application: 01.08.2007
(73) Proprietor: Lonza AG, 4052 Basel (CH)
(72) Inventor: QUATTRINI, Francesca, CH-3904 Naters (CH); VARRAY, Stéphane, CH-3960 Sierre (CH); WERBITZKY, Oleg, CH-3968 Veyras (CH); ZEITER, Thomas, CH-3932 Visperterminen (CH)
(74) Representative: Riegler, Norbert Hermann
(86) International application number: PCT/EP2005/011383
(87) International publication number: WO 2006/048144

(56) References cited:
- EP-A- 1 164 143
- CAI R-Z ET AL: "SYNTHESIS AND BIOLOGICAL ACIVITY OF HIGHLY POTENT OCTAPEPTIDE ANALOGS OF SOMATOSTATIN" PROCEEDINGS OF THE NATIONAL ACADEMY OF SCIENCES OF USA, NATIONAL ACADEMY OF SCIENCE, WASHINGTON, DC, US, vol. 83, March 1986 (1986-03), pages 1896-1900, XP009010969 ISSN: 0027-8424
- VOLKMER-ENGERT R ET AL: "CHARCOAL SURFACE-ASSISTED CATALYSIS OF INTRAMOLECULAR DISULFIDE BOND FORMATION IN PEPTIDES" JOURNAL OF PEPTIDE RESEARCH, MUNKSGAARD INTERNATIONAL PUBLISHERS, COPENHAGEN, DK, vol. 51, no. 5, May 1998 (1998-05), pages 365-369, XP000739870 ISSN: 1397-002X cited in the application

## Description

The present invention relates to an improved method for disulfide-bonding driven cyclization of a peptide.

Vapreotide is a cyclic octapeptide comprising one cystine moiety. It is a synthetic analogue of somatostatin which hormone inhibits secretion of hormones such as insulin and glucagon, important regulators of fat and sugar metabolism and growth hormone. Somatostatin got a physiological half-life of 3 minutes, whereas is apreotide is much long-lasting in the circulation, owing to C-terminal amidation of the analogue. Disulfide bonding is a feature that is paramount for activity both of vapreotide and somatostatin.

Somatostatin has the structure:

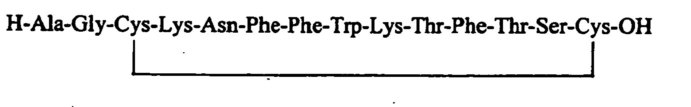
H-Ala-Gly-Cys-Lys-Asn-Phe-Phe-Trp-Lys-Thr-Phe-Thr-Ser-Cys-OH

Vapreotide has the structure:

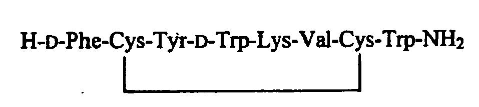
H-D-Phe-Cys-Tyr-D-Trp-Lys-Val-Cys-Trp-NH2


Volkmer-Engert et al. (Surface-assisted catalysis of intramolecular disulfide bond formation in peptides , J. Peptide Res. 51, 1998, 365-369) describe charcoal-catalyzed oxidative formation of intramolecular disulfide bonds in water by using oxygen dissolved in the solvent, i.e. water. Careful controls showed that the pool of oxygen physically dissolved in the aequeous medium was necessary and sufficient to load the charcoal with oxygen for oxidation. Use of charcoal, as compared to traditional air-sparging in the absence of catalyst, accelerated the reaction rate for intramolecular cystine formation dramatically and selectively.

The use of charcoal as a heterogeneous catalyst inevitably requires to carry out such reaction in solution but not on-resin. It has become a widely applied method where applicable; the method imposes some restrictions on the solvent system not suitable for every peptide.

Cai et al. (Proc. Natl. Acad. Sci. USA, 1986, 83, 1896-1900) describe the cyclization of vapreotide in presence of ferricyanide or iodine affording the poor yields of 7% and 25% respectively.

US 6476186 devises intramolecular disulfide bonding of the linear, deprotected octapeptide H-D-Phe-Cys-Phe-D-Trp-Lys-Thr-Cys-Thr-OH in acetonitrile water (1:1) in the presence of trace amounts of charcoal. The peptide was synthesized on 2-chlorotrityl resin and comprises apart from hydrophobic residues and the cysteines, a lysine and a threonine. Cysteines were protected with acid-labile trityl groups. Charcoal-catalyzed cyclization took place after cleavage and deprotection in the aqueous solvent mix, carefully adjusted to pH 8.0 with sodium hydroxide, for 5 hours at a concentration of 50 mg/mL. Yield of pure product was about 80%.

As a disadvantage, when seeking to apply the same methodology to non-cyclic vapreotide which is a less water-soluble peptide amide in contrast, 80% analytical yield was achievable but only after 44 h of extended reaction time and at a dilution of about 1 mg/mL. Elevated concentrations of only 5-10 mg/mL gave rise to slow precipitation of educt, lowering the product yield accordingly. Higher permanent concentrations of about 10 mg/mL could be achieved by change to pH 6, but ensued formation of a side product, hence reducing yield to 30%. For an efficient industrial-scale process, much shorter reaction times with higher through-put of material are needed.

It is an object of the present invention to devise another or improved method for cyclization of the linear peptide precursor of mature vapreotide, avoiding the disadvantages of the prior art. This object is solved by the present method of cyclisizing a linear peptide H-D-Phe-Cys-Tyr-D-Trp-Lys-Val-Cys-Trp-NH₂ by means of intramolecular cystine formation, comprising the step ofcyclisizing said peptide at a concentration of at least 20 mg/mL in a polar, aprotic solvent and in the presence of charcoal, in the further presence of a primary, secondary or tertiary amine as a base reagent, and of an oxidizing reagent.

The afore said peptide may be produced by any method known in the art, be it biotechnologically or by means of liquid or solid phase chemical synthesis. Methods of solid-phase synthesis are described in Bodanzky, M., Principles of Peptide Synthesis, 2nd ed. Springer Verlag Berlin/Heidelberg, 1993, for instance. C-terminal amidation may be achieved by enzymatic or chemical methods well-known in the art, e.g. by solid-phase synthesis on a resin in C-terminal linkage on well-known Sieber or Rink amide resin, giving rise to carboxamide upon cleavage from resin. Chemical synthesis usually requires use of protecting groups for individual amino acids as is routinely known in the art. A large variety of protecting groups can be employed for protection of cysteine residues, e.g. trityl, acetamidomethyl (Acm), t-butyl, trimethylacetamidomethyl, 2,4,6-trimethoxybenzyl, methoxytrityl, t-butylsulphenyl. Removal of such protecting groups will require specific reagents or reaction conditions, depending on the type of protecting group employed.

Most commonly, the trityl group is employed for simple protection during peptide synthesis and may then be removed by simple acidolysis e.g. in 30-60% TFA concomitant with other protecting groups and the resin linker. For the present invention, it is only needed that the cysteiny-containing peptide sought to be cyclizised has free, unprotected cysteines for applying the method of the present invention. As a side issue, it may be helpful to mention that Atherton et al. (1985, J. Chem. Perkin Trans. I., 2065) reported that upon acidolysis, the use of the popular scavenger thioanisole which is bifunctional in being both scavenger and also acidolysis promoter also resulted in partial deprotection of Acm, t-butyl and S-t-butylsulphenyl protected cysteines. This would also allow of working the present invention. More quantitatively, S-t-butylsulphenyl groups are easily and selectively removable under mild reaction conditions by treatment with thiol reagents such as beta-mercaptoethanol or dithiothreitol, or by treatment with e.g. triphenyl- or triethylphosphine. In contrast, customarily in the art Acm-protecting groups are quantitatively removed by iodine oxidation concomitant with cyclization; hence iodine oxidation-borne deprotection of Acm groups is not compatible with the present invention.

The polar, aprotic solvent preferably is a solvent miscible with both water and acetone. Suitable examples of solvents of this type in accordance with the present invention are e.g. dimethylformamide (DMF), N-methylpyrrolidone (NMP), acetamide or tetrahydrofuran (THF). It goes without saying that a suitable solvent in the present context necessarily is compliant with achieving a peptide concentration of at least 20 mg/mL. Consequently, acetonitrile is not a suitable solvent according to the present invention as is shown in the comparative examples of the experimental section. Preferably, the solvent is selected from the group consisting of acetamide, dimethylformamide and N-methylpyrrolidone.

More preferably, the solvent is selected from the group consisting of dimethylformamide and N-methylpyrrolidone. Most preferably, the solvent is dimethylformamide.

Preferably, the base is a weak base of a conjugated pKa of from 7.5 to 10, and is a secondary, more preferably a sterically hindered, tertiary amine. Examples of such, and further preferred, are Hünig-base (N,N-diisopropylethylamine), N,N'-dialkylaniline, 2,4,6-trialkylpyridine or N-alkylmorpholine with the alkyl being straight or branched C₁-C₄ alkyl, more preferably it is N-methylmorpholine or collidine (2,4,6-trimethylpyridine), most preferably it is collidine.

The oxidizing reagent preferably is air and/or oxygen. When switching from aqueous solvent system of the prior art to the non-aqueous solvent system of the present invention, it is strongly preferred to ensure saturation of the catalyst with oxygen by actively supplying or ventilating the solvent liquid with air and/or oxygen, at least during a period of time, e.g. by directing a steady or timewise stream of air from a submerged gas sparger into the solvent. Optionally, a strong vortex arising from stirring of the solution along with a favorable aspect ratio of the liquid phase relative to the vortex in order to avoid local mixing only, and with possibly suitably shaped, e.g. propeller-like or bead - like stirrer device or stirring means, can be used.

The charcoal or activated charcoal may be of the same type as employed in the prior art. Preferably, it is used in at least catalytic amounts. It goes without saying that the reaction rate is both influenced by the amount of catalyst present and by the concentration of starting materials, the latter enhancing the absorption rate to or the loading of the solid surface of the heterogeneous catalyst with cysteinyl-containing peptide from the solution. However, according to the present invention, the choice of solvent strongly contributes to efficient catalysis. Further it was observed, without wanting to be very strictly bound to such observation or any respective theory, that the reaction kinetics were depending not only on the solvent system u but also on the amount of starting material: Whilst increasing the amount of starting material the range of 1 to 100 mg/mL, the reaction kinetics display an entirely surprising change in shape and become ever more linear rather than sigmoidal. Some yet unreported transition phenomenon at the phase border solid-liquid might account for that. In result, 100% conversion rates amounting to even somewhat improved product yields were achievable at dramatically shorter reaction times by increasing the concentration of the starting material. This has not previously been accounted for when using charcoal-catalyzed oxidation. Therefore the afore cysteinyl-containing peptide-precursor H-D-Phe-Cys-Tyr-D-Trp-Lys-Val-Cys-Trp-NH₂, is added to the reaction in an amount of at least 20 mg/mL, more preferably 50 mg/mL, most preferably 100 mg/mL according to the present invention. The upper limit is given by the maximum solubility of course.

The cyclization reaction may be carried out under refluxing conditions at a temperature rage of from about 0°C to 80°C, more preferably of from 5°C to 60°C, most preferably at about 15 to 40°C, and preferably in conjunction with using air/oxygen as oxidizing reagent.

### Experiments

All cyclisation reactions were carried out at 25°C.Vapreotide product of formula I 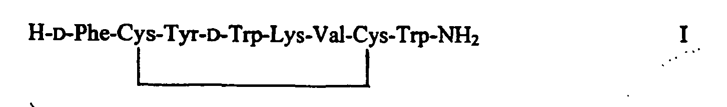 was always purified by directly loading the reaction mixture onto reverse-phase HPLC.

### 1.Synthesis of H-D-Phe-Cys-Tyr-D-Trp-Lys-Val-Cys-Trp-NH₂

The linear peptide was synthesized on Sieber-resin (from Novabiochem) by Fmoc standard methods. The side chain protecting groups were D- or L-Trp(Boc), Cys(Trt), Lys(Boc), Tyr(tBu). The protected peptide was cleaved off by 5% TFA in dichloromethane and then globally deprotected by acidolysis in a cleavage mixture of (eq.= volume parts) 300 eq. conc. TFA, 12 eq. dithiothreitol, 12. eq. dichloromethane, 50 eq. water (aqua dest) at 1 h at room temperature. The product was precipitated by adding methyl-t-butyl-ether, complete removal of Boc groups in the product H-D-Phe-Cys-Tyr-D-Trp-Lys-Val-Cys-Trp-NH₂ and the purity was verified by HPLC.

### 2. Comparative example: Cyclisation of H-D-Phe-Cys-Tyr-D-Trp-Lys-Val-Cys-Trp-NH₂

The product from example 1 was subjected to the charcoal method essentially as described in US 6476186 and using acetonitrile: water (1:1), except that a concentration of the linear cysteinyl-containing peptide of 1mg/mL was used, the pH was adjusted to about pH 8 with ammonia and that additionally air was sparged at low pressure into the liquid unter stirring. After 44 h, 100% conversion was achieved, with 84% (w/w) analytical yield after immediate HPLC purification of the product from the reaction mixture. Higher concentrations of up to 10 mg/mL were found to be unstable and the starting material would have started to precipitate after a short time. Solubility of the peptide could also not be significantly improved by increasing the ratio of acetonitrile: water to 3-2:1; rather reaction time at 1 mg/mL increased by 50-100%, with concomitant loss of purity (yield: 74% (w/w)).

### 3. Comparative example : Cyclisation of H-D-Phe-Cys-Tyr-D-Trp-Lys-Val-Cys-Trp-NH₂

The reaction of example 2 was repeated with the sole difference that ammonia/NH₄Cl was used to adjust the pH to 6 and to dissolve the cysteinyl-containing peptide at 10 mg/mL accordingly. After 70 hours, a conversion rate of 80% was reached whilst the product had only low purity. Yield of product I was 31 %.

### 4. Cyclisation of H-D-Pbe-Cys-Tyr-D-Trp-Lys-Val-Cys-Trp-NH₂

The product from example 1 was subjected to the charcoal method using trace amounts of activated, powdered charcoal, a concentration of the linear cysteinyl-containing peptide of 50 mg/mL (1 eq.) in dimethylformamide in the presence of 1 eq. diisopropyl-ethyl-amine and that additionally air was sparged at low pressure into the liquid unter stirring.

After 15-20 hours, 100% conversion was achieved with an analytical yield of 79%. The experiment was conducted thrice independently, always with the same result.

## Claims

1. Method for cyclization of the linear peptide H-D-Phe-Cys-Tyr-D-Trp-Lys-Val-Cys-Trp-NH₂ by means of intramolecular cystine formation, comprising the step of cyclisizing said peptide at a concentration of at least 20 mg/mL in a polar, aprotic solvent selected from the group consisting of dimethylformamide, N-methyl-pyrrolidone, acetamide and tetrahydrofuran, and in the presence of charcoal, in the further presence of a primary, secondary or tertiary amine as a base reagent and of an oxidizing reagent.

2. Method according to claim 1, **characterized in that** the charcoal is present in at least catalytic amount.

3. Method according to claim 1, **characterized in that** the oxidizing reagent is air and/or oxygen.

4. Method according to claim 3, **characterized in that** the reaction is supplied with air and/or oxygen during the reaction.

5. Method according to claim 4, **characterized in that** the reaction vessel has means to direct the air and/or oxygen directly into the liquid, preferably is comprising multiple orifices in the bottom and/or walls of a reaction vessel or is comprising at least one submerged gas sparger, from which means or sparger air is bubbling directly into the solvent.

6. Method according to one of the preceding claims, **characterized in that** the solvent is selected from the group consisting of dimethylformamide and N-methylpyrrolidone.

7. Method according to claim 6, **characterized in that** the solvent is dimethylformamide.

8. Method according to claim 1, **characterized in that** the concentration of the peptide is at least 50 mg/mL, preferably that the solvent is dimethylformamide and that the concentration of the peptide is at least 100 mg/mL.

## Patentansprüche

1. Verfahren zur Cyclisierung des linearen Peptids H-D-Phe-Cys-Tyr-D-Trp-Lys-Val-Cys-Trp-NH₂ durch intramolekulare Cystinbildung, bei dem man das Peptid bei einer Konzentration von mindestens 20 mg/mL in einem polaren aprotischen Lösungsmittel aus der Gruppe bestehend aus Dimethylformamid, *N*-Methylpyrrolidon, Acetamid und Tetrahydrofuran in Gegenwart von Aktivkohle und in weiterer Gegenwart eines primären, sekundären oder tertiären Amins als Basenreagens und eines Oxidationsmittels cyclisiert.

2. Verfahren nach Anspruch 1, **dadurch gekennzeichnet, dass** die Aktivkohle in mindestens katalytisch wirksamer Menge vorliegt.

3. Verfahren nach Anspruch 1, **dadurch gekennzeichnet, dass** es sich bei dem Oxidationsmittel um Luft und/oder Sauerstoff handelt.

4. Verfahren nach Anspruch 3, **dadurch gekennzeichnet, dass** man dem Ansatz während der Umsetzung Luft und/oder Sauerstoff zuführt.

5. Verfahren nach Anspruch 4, **dadurch gekennzeichnet, dass** das Reaktionsgefäß Mittel aufweist, um die Luft und/oder den Sauerstoffs direkt in die Flüssigkeit zu leiten, und vorzugsweise eine Vielzahl von Öffnungen im Boden und/oder den Wänden des Reaktionsgefäßes oder mindestens einen untergetauchten Gasverteiler umfasst, wobei von dem Mittel oder Verteiler Luft direkt in das Lösungsmittel perlt.

6. Verfahren nach einem der vorhergehenden Ansprüche, **dadurch gekennzeichnet, dass** man das Lösungsmittel aus der Gruppe bestehend aus Dimethylformamid und Mmethylpyrrolidon auswählt.

7. Verfahren nach Anspruch 6, **dadurch gekennzeichnet, dass** es sich bei dem Lösungsmittel um Dimethylformamid handelt.

8. Verfahren nach Anspruch 1, **dadurch gekennzeichnet, dass** die Konzentration des Peptids mindestens 50 mg/mL beträgt, wobei vorzugsweise das Lösungsmittel Dimethylformamid ist und die Peptidkonzentration mindestens 100 mg/mL beträgt.

## Revendications

1. Méthode de cyclisation du peptide linéaire H-D-Phe-Cys-Tyr-D-Trp-Lys-Val-Cys-Trp-NH₂ par formation de cystine intramoléculaire, comprenant l'étape de cyclisation dudit peptide à une concentration au moins égale à 20 mg/mL dans un solvant polaire aprotique choisi dans le groupe constitué du diméthylformamide, de la *N*-méthylpyrrolidone, de l'acétamide et du tétrahydrofurane, et en présence de charbon activé, en présence en outre d'une amine primaire, secondaire ou tertiaire en tant que réactif basique et d'un réactif oxydant.

2. Méthode selon la revendication 1, **caractérisée en ce que** le charbon activé est présent au moins en une quantité catalytique.

3. Méthode selon la revendication 1, **caractérisée en ce que** le réactif oxydant est l'air et/ou l'oxygène.

4. Méthode selon la revendication 3, **caractérisée en ce que** la réaction est alimentée en air et/ou en oxygène au cours de la réaction.

5. Méthode selon la revendication 4, **caractérisée en ce que** la cuve réactionnelle est équipée de moyens permettant de diriger l'air et/ou l'oxygène directement dans le liquide, de préférence comprend de multiples orifices au fond et/ou dans les parois d'une cuve réactionnelle ou comprend au moins un distributeur de gaz immergé, moyens ou distributeur à partir desquels ou duquel l'air barbote directement dans le solvant.

6. Méthode selon l'une des revendications précédentes, **caractérisée en ce que** le solvant est choisi dans le groupe constitué du diméthylformamide et de la *N*-méthylpyrrolidone.

7. Méthode selon la revendication 6, **caractérisée en ce que** le solvant est le diméthylformamide.

8. Méthode selon la revendication 1, **caractérisée en ce que** la concentration du peptide est au moins égale à 50 mg/mL, de préférence **en ce que** le solvant est le diméthylformamide et **en ce que** la concentration du peptide est au moins égale à 100 mg/mL.
